(19) Europäisches Patentamt
European Patent Office
Office européen des brevets

(11) Veröffentlichungsnummer : **0 025 109**
**B1**

(12) # EUROPÄISCHE PATENTSCHRIFT

(45) Veröffentlichungstag der Patentschrift : **15.05.85**

(51) Int. Cl.⁴ : **C 07 D313/08**, A 61 K 31/335

(21) Anmeldenummer : **80104466.0**

(22) Anmeldetag : **29.07.80**

(54) **1-Benzoxepin-5(2H)-on-Derivate und ihre Salze sowie Verfahren zu deren Herstellung.**

(30) Priorität : **02.08.79 DE 2931398**

(43) Veröffentlichungstag der Anmeldung : **18.03.81 Patentblatt 81/11**

(45) Bekanntmachung des Hinweises auf die Patenterteilung : **15.05.85 Patentblatt 85/20**

(84) Benannte Vertragsstaaten : **AT BE CH DE FR GB IT LI LU NL SE**

(56) Entgegenhaltungen :
**DE-A- 1 593 760**
**Chemical Abstract Band 66, Nr. 5, 30. Januar 1967 Columbus, Ohio, USA J.H.P. TYMAN et al. "Novel preparation of 1-benzoxepins" Seite 1801, Spalte 1, Abstract Nr. 18659u**
**THE JOURNAL OF ORGANIC CHEMISTRY, Band 42, Nr. 25, 09. Dezember 1977 B.K. WASSON et al. "A Synthesis of 6-Hydroxy-1-Benzoxepin-3,5(2H,4H)-Dione" Seiten 4265 bis 4266**
**Die Akte enthält technische Angaben, die nach dem Eingang der Anmeldung eingereicht wurden und die nicht in dieser Patentschrift enthalten sind.**

(73) Patentinhaber : **Kali-Chemie Pharma GmbH**
**Hans-Böckler-Allee 20 Postfach 220**
**D-3000 Hannover 1 (DE)**

(72) Erfinder : **Ohlendorf, Heinrich-Wilhelm Dipl.-Chem. Dr.**
**Lilienstrasse 19**
**D-3000 Hannover 1 (DE)**
Erfinder : **Wolf, Klaus-Ullrich, Dr.**
**Imkersweg 6**
**D-3165 Hänigsen (DE)**
Erfinder : **Kaupmann, Wilhelm Dipl.-Chem. Dr.Ing.**
**St. Ingbert-Weg 9**
**D-3000 Hannover-Kirchrode (DE)**
Erfinder : **Heinemann, Henning Dipl.-Chem. Dr.rer.nat.**
**Bergiusstrasse 18**
**D-3000 Hannover 51 (DE)**

(74) Vertreter : **Lauer, Dieter, Dr. et al**
**c/o Kali-Chemie Aktiengesellschaft Postfach 220**
**D-3000 Hannover 1 (DE)**

**EP 0 025 109 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 1-Benzoxepin-5(2H)-on-Derivate und ihre Salze, Verfahren zu deren Herstellung sowie diese Substanzen enthaltende pharmazeutische Zusammensetzungen.

Der vorliegenden Erfindung liegt die Aufgabe zugrunde, neue Verbindungen mit wertvollen pharmakologischen und therapeutischen Eigenschaften herzustellen.

Überraschenderweise wurde gefunden, daß die neuen 3-Amino-1-benzoxepin-5(2H)-on-Derivate eine gute Wirkung auf die Motilität des Magens haben.

Gegenstand der Erfindung sind daher neue 3-Amino-1-benzoxepin-5(2H)-on-Derivate der Formel I

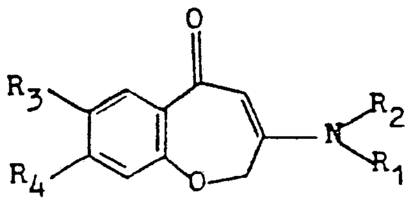

worin $R_1$ und $R_2$ unabhängig voneinander jeweils ein Wasserstoffatom, eine $C_1$-$C_5$-Alkylgruppe, welche gegebenenfalls einen unsubstituierten Phenylrest oder einen durch ein oder zwei Halogenatome, Methyl- oder Methoxygruppen oder eine 3,4-Methylendioxy- oder 3,4-Äthylendioxygruppe substituierten Phenylrest endständig tragen kann, eine gegebenenfalls endständig mit einer Hydroxy- oder Methoxygruppe substituierte $C_2$-$C_5$-Alkylgruppe oder eine $C_3$-$C_4$-Alkenylgruppe bedeuten, oder einer der beiden Reste $R_1$ und $R_2$ ein Wasserstoffatom oder eine $C_1$-$C_5$-Alkylgruppe und der andere eine $C_2$-$C_5$-Alkylgruppe, endständig substituiert mit einer $NR_5R_6$-Gruppe — in welcher $R_5$ und $R_6$ unabhängig voneinander jeweils ein Wasserstoffatom oder eine $C_1$-$C_5$-Alkylgruppe sind oder in welcher Alkylgruppen $R_5$ und $R_6$ direkt oder über ein Heteroatom O, S oder $NR_7$ unter Bildung eines 5- bis 7-Ringes miteinander verbunden sind — bedeuten, oder die Alkylgruppen $R_1$ und $R_2$ direkt oder über ein Heteroatom O, S oder $NR_7$ unter Bildung eines 5- bis 7-Ringes miteinander verbunden sind, wobei $R_7$ ein Wasserstoffatom, eine Methyl-, Benzyl- oder Phenylgruppe ist, und worin $R_3$ und $R_4$ unabhängig voneinander jeweils Wasserstoffatom, Halogenatom, Alkyl-, Alkoxy- oder Alkylthiogruppe bedeuten, wobei der Alkylrest jeweils 1 bis 4 Kohlenstoffatome haben kann, oder einer der beiden Reste $R_3$ und $R_4$ Trifluor-methyl- oder Nitrogruppe und der andere Wasserstoffatom sind, sowie Säureadditionsalze solcher Verbindungen der Formel I, worin $R_1$ und/oder $R_2$ ein Stickstoffatom enthalten.

Als gegebenenfalls substituierte Alkylreste bzw. Alkenylreste $R_1$ und $R_2$ kommen gerade oder verzweigte Gruppen mit bis zu 5 Kohlenstoffatomen in Frage, so beispielsweise die Methyl-, Äthyl-, Propyl-, Isopropyl-, Butyl-, Isobutyl-, tert.-Butyl-, Pentyl-, Isopentyl-, Neopentyl-, Allyl-, 2-Butenyl- oder 3-Butenylreste. Bevorzugt sind Verbindungen, in welchen nur einer der Reste $R_1$ und $R_2$ einen substituierten Alkylrest bedeutet und der andere ein Wasserstoffatom oder Alkylrest ist.

Für $R_5$ une $R_6$ kommen die obengenannten Alkylgruppen mit 1 bis 5 Kohlenstoffatomen in Frage.

Wenn die Alkylgruppen an den Stickstoffatomen entweder direkt oder über ein Heteroatom miteinander verbunden sind, kommen z. B. in Frage Pyrrolidin, Piperidin, Azacycloheptan, Morpholin, Thiomorpholin, Piperazin und Homopiperazin, wobei letztere gegebenenfalls am Stickstoffatom mit Methyl, Benzyl oder Phenyl substituiert sein können. Bevorzugt werden die Verbindungen mit 5- bzw. 6-Ring.

Für die Substituenten $R_3$ und $R_4$ am Phenylring kommen als Halogenatome Fluor-, Chlor-, Brom- oder Jodatome, insbesondere Fluor-, Chlor- oder Bromatome in Frage. Die $C_1$- bis $C_4$-Alkylreste in den Alkyl-, Alkoxy- oder Alkylthiogruppen können gerade oder verzweigt sein, wobei insbesondere bei zweifacher Substitution am Phenylring der Methylrest, so Methyl, Methoxy oder Methylthio, vorherrscht. Für die Nitro- oder Trifluormethylgruppe ist die Monosubstitution vorgesehen.

Bei Substitution mit gegebenenfalls substituiertem Piperazin oder Homopiperazin bzw. Alkyl-$NR_5R_6$ der oben angegebenen Bedeutung, können die aus dem Reaktionsgemisch isolierten freien Basen gewünschtenfalls durch Umsetzung mit anorganischen oder organischen Säuren nach an sich bekannter Weise in ihre physiologisch verträglichen Säureadditionssalze überführt werden. Als geeignete Säuren haben sich beispielsweise Chlorwasserstoffsäure, Bromwasserstoffsäure, Schwefelsäure, Salpetersäure, Orthophosphorsäure, Maleinsäure, Cyclohexylaminosulfonsäure, Amidosulfonsäure oder p-Toluolsulfonsäure erwiesen.

Gegenstand der Erfindung ist ferner ein Verfahren zur Herstellung der 3-Amino-1-benzoxepin-5(2H)-on-Derivate der Formel I

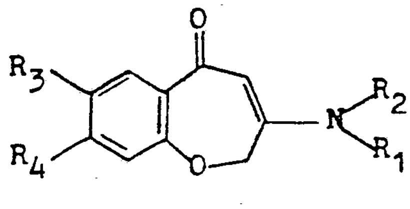

worin $R_1$, $R_2$, $R_3$ und $R_4$ die obige Bedeutung haben, sowie deren Säureadditionssalzen, dadurch gekennzeichnet, daß man eine Verbindung der Formel II

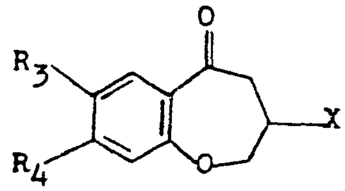

worin $R_3$ und $R_4$ die obige Bedeutung haben, oder eine Verbindung der Formel IV

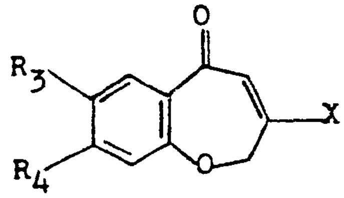

worin $R_3$ und $R_4$ die obige Bedeutung haben und X Chlor oder Brom ist, mit einem Amin der Formel III

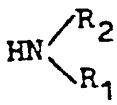

worin $R_1$ und $R_2$ die obige Bedeutung haben, in einem inerten Lösungsmittel umsetzt, die freie Base isoliert und sie gegebenenfalls in ein Säureadditionssalz überführt oder aus deren Säureadditionssalz die freie Base isoliert.

Die Umsetzung einer Verbindung II oder IV mit einem Amin der Formel III kann in an sich bekannter Weise erfolgen. Die Umsetzung der 2,3,4,5-Tetrahydro-1-benzoxepin-3,5-dion-Derivate der Formel II mit den der Formel III kann durch Zugabe katalytischer Mengen an anorganischen oder organischen Säuren, wie beispielsweise Salzsäure, Schwefelsäure, p-Toluolsulfonsäure oder Ameisensäure, begünstigt werden. Als inerte Lösungsmittel können beispielsweise Chloroform, Dichlormethan, Benzol oder Toluol dienen. Die Reaktion kann im Temperaturbereich von 0 bis 150 °C durchgeführt werden. Die Umsetzung kann dadurch verbessert werden, daß man das sich bildende Wasser während der Reaktion auf übliche Weise entfernt. Bei Verwendung der Verbindungen der Formel IV kann man die Umsetzung mit dem Amin der Formel III in einem inerten Lösungsmittel, wie Chloroform, Dichlormethan, Dimethylformamid, Dioxan oder Tetrahydrofuran, bei Temperaturen zwischen − 70 und + 50 °C, durchführen, wobei die Reaktion vorzugsweise in Gegenwart einer organischen Base, wie Triäthylamin, oder einer überschüssigen Menge des eingesetzten Amins vorgenommen wird.

Die neuen Verbindungen der Formel IV lassen sich in an sich bekannter Weise dadurch herstellen, daß man die 2,3,4,5-Tetrahydro-1-benzoxepin-3,5-dion-Derivate der Formel II mit einem entsprechenden Säurehalogenid umsetzt. Als Säurehalogenide kommen Phosphoroxidhalogenide, Phosphortrihalogenide, Thionylchlorid oder insbesondere Oxalylchlorid in Frage. In Gegenwart eines inerten Lösungsmittels, beispielsweise Dichlormethan oder Dimethylformamid, kann die Umsetzung im Temperaturbereich von − 20 bis 80 °C durchgeführt werden. Für die Umsetzung mit dem Amin der Formel III kann das vom überschüssigen Säurehalogenid und vom Lösungsmitel befreite Reaktionsprodukt eingesetzt werden.

Die als Ausgangsverbindungen eingesetzten 2,3,4,5-Tetrahydro-1-benzoxepin-3,5-dion-Derivate der Formel II

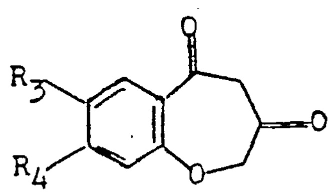

in welcher $R_3$ und $R_4$ die obige Bedeutung haben, können dadurch hergestellt werden, daß man Verbindungen der Formel V

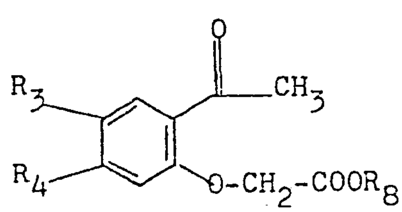

in welcher $R_3$ und $R_4$ die obige Bedeutung haben und $R_8$ eine gerade oder verzweigte niedermolekulare Alkylgruppe, vorzugsweise eine Methylgruppe, ist, mit einer starken Base aus der Reihe Lithiumhydrid, Natriumhydrid, Lithiumtert.-butylat oder Kalium-tert.-butylat in Gegenwart eines inerten Lösungsmittels bei Temperaturen zwischen − 70 °C und der Siedetempereatur des Lösungsmittels umsetzt. Als Lösungsmittel eignen sich beispielsweise Dimethylformamid oder Tetrahydrofuran.

Zur Aufarbeitung kann das Reaktionsgemisch mit Eiswasser, versetzt und die ausfallende Verbindung der Formel II abgetrennt werden. Man kann aber auch durch Ausfällen der Alkalisalze, insbesondere des Lithiumsalzes, mit einem unpolaren Lösungsmittel, beispielsweise Toluol oder Petroläther, die Verbindungen der Formel II von den Nebenprodukten abtrennen. Aus dem Salz kann die freie Verbindung mittels einer anorganischen oder organischen Säure, beispielsweise einer wäßrigen Lösung von Salzsäure, Schwefelsäure oder Essigsäure, freigesetzt werden.

Es ist überraschend, daß unter Verwendung der oben genannten Basen, vorzugsweise Natriumhydrid und Lithiumtert.-butylat, die Verbindungen der Formel II unter Ringschluß der Verbindungen der Formel V in guten Ausbeuten erhalten werden können. Es ist nämlich bekannt, daß bei den Cyclisierungsversuchen von 2'-Acetylphenoxyacetaten mit dem üblicherweise eingesetzten Natriumäthylat Benzofuranderivate als Hauptreaktionsprodukte erhalten werden und Benzoxepin-3,5-dione gar nicht oder nur in geringen Mengen als Nebenprodukte erhalten werden (s. J. Org. Chem. Vol. 42 (1977) S. 4265 sowie Tetrahedron Letters (1966) No. 41, S. 4995, Abs. 1). Die für die Herstellung von 2,3,4,5-Tetrahydro-1-benzoxepin-3,5-dion-Derivaten der Formel II erforderlichen 2'-Acetylphenoxyacetate können bekanntlich aus den 2-Hydroxyacetophenon in guter Ausbeute erhalten werden, so daß ein einfacher Weg zur Herstellung des pharmakologisch interessanten 3-Amino-1-benzoxepin-5(2H)-on-Derivate der Formel I gegeben ist.

Die neuen Verbindungen und ihre Säureadditionssalze weisen wertvolle therapeutische Eigenschaften auf, vor allem zeigen sie eine hemmende und regulierende Wirkung bei Spasmen der glatten Muskulatur im Magen-Darm-Kanal.

Schmerzhafte abdominale Erkrankungen werden häufig durch Spasmen der gastrointestinalen Muskulatur hervorgerufen. Sie sind das therapeutische Ziel einer Vielzahl spasmolytisch wirkender Medikamente. Vor allem die Parasympathicolytica werden schon seit geraumer Zeit angewandt. Die mangelhafte Selektivität ihrer Wirkung setzt jedoch ihrem Einsatz enge Grenzen. Vor allem Mundtrockenheit, Sehstörungen und Harnretention verbieten die Applikation ausreichend hoher Dosen.

Überraschenderweise wurde gefunden, daß die erfindungsgemäßen Substanzen spezifisch die gewünschte hemmende und regulierende Wirkung zeigen, ohne daß chemische oder pharmakologische Hinweise auf die oben genannten Nebenwirkungen gegeben sind.

## Beschreibung der pharmakologischen Untersuchungsmethoden

### 1. Akute Toxizität

Die akute 7-Tage-Toxizität wird nach einmaliger Applikation intraperitoneal an der weißen nüchternen NMRI-Maus bestimmt. Die Berechnung der $LD_{50}$-Werte erfolgt über EDV durch eine Probitanalyse (L. Cavalli-Sforza, Gustav Fischer-Verlag, Stuttgart (1964), Grundbegriffe der Biometrie, S. 153 ff.).

### 2. Prüfung der Magenperistaltik

Zur Bestimmung der Magenperistaltik werden etwa 200 g schweren, mit Ketamino-Hydrochlorid/Xylazin narkotisierten Ratten in die Vena jugularis ein Gefäß-Katheter und in die Trachea ein Trachealkatheter eingeführt. In den Magen wird eine Magensonde eingebunden, die über einen Dreiwegehahn mit einem Statham-Druckgeber (P 23 DB) verbunden wird. Der Magen wird am Pylorus und an der Cardia durch eine Ligatur verschlossen. Anschließend wird der Magen mit 3 ml 0,9 %-iger NaCl gefüllt. Die vom Magen erzeugten Drucke werden kontinuierlich von einem Watanabe-Multicorder (MC 641) registriert.

Zur Durchführung der Versuche wird eine Stimulation der peristaltischen Bewegungen des Magens durch Dauerinfusion von 50 mg/kg/h Bariumchlorid i. v. vorgenommen und die auftretenden Amplituden und Frequenzen der vom Magen erzeugten Druckwellen gemessen (Kontrollwerte), Anschließend werden die Testsubstanzen in physiologischer Natriumchloridlösung gelöst oder in Tylose MH50 suspendiert in einer Dosis von 20 mg/kg intraperitoneal appliziert und die Amplituden- und Frequenzänderung der Druckwellen ermittelt.

Die Auswertung ergibt, daß kurz nach Applikation der erfindungsgemäßen Substanzen eine Herabsetzung der peristaltischen Bewegungen des Magens erfolgt, die sich in einer deutlichen Senkung der Amplituden bemerkbar macht. Die Frequenz wird nur im geringeren Maße verändert, wie der nachfolgenden Tabelle zu entnehmen ist. Die geringe Toxizität der Substanzen gewährleistet eine gute Verträglichkeit derselben. Ein weiterer Vorteil ist der beobachtete rasche Wirkungseintritt.

Nach den beschriebenen Methoden wurden beispielsweise folgende Substanzen untersucht:

A) 3-Methylamino-1-benzoxepin-5(2H)-on
B) 3-Methylamino-8-chlor-1-benzoxepin-5(2H)-on

C) 3-Isopropylamino-1-benzoxepin-5(2H)-on
D) 3-Amino-1-benzoxepin-5(2H)-on
E) 3-(n-Butylamino)-1-benzoxepin-5(2H)-on
F) 3-Phenäthylamino-1-benzoxepin-5(2H)-on
G) 3(N-Benzylpiperazino)-1-benzoxepin-5(2H)-on
H) 3-Morpholino-1-benzoxepin-5(2H)-on
I) 3-(ß-Methoxyäthylamino)-1-benzoxepin-5(2H)-on
K) 3-Methylamino-8-methyl-1-benzoxepin-5(2H)-on
L) 3-Methylamino-7-methyl-1-benzoxepin-5(2H)-on
M) 3-Methylamino-8-tert.-butyl-1-benzoxepin-5(2H)-on
N) 3-Methylamino-7-chlor-1-benzoxepin-5(2H)-on
O) 3-Methylamino-7-fluor-1-benzoxepin-5(2H)-on

Tabelle

| Substanzen | Senkung der Amplitude in % | Frequenzänderung in % | $LD_{50}$ i.p. (mg/kg) |
|---|---|---|---|
| A | 56 | - 18 | 664 |
| B | 58 | - 16 | 450 |
| C | 32 | - 2 | 544 |
| D | 45 | - 12 | 544 |
| E | 26 | + 36 | 442 |
| F | 29 | - 7 | n.b. |
| G | 31 | + 10 | 634 |
| H | 12 | + 17 | 650 |
| I | 46 | + 11 | n.b. |
| K | 55 | + 4 | n.b. |
| L | 75 | + 15 | n.b. |
| M | 72 | + 10 | n.b. |
| N | 46 | - 1 | n.b. |
| O | 37 | + 1 | n.b. |

n.b. = nicht bestimmt

Die pharmakologisch beobachteten Wirkungen lassen eine günstige Beeinflussung bei verschiedenen spastischen Zuständen sowohl im Magen-Darm-Kanal als auch im Gallenwegsystem am Menschen erwarten.

Die Arzneimittelzubereitungen enthalten die Substanzen der Formel I oder deren pharmakologisch verträgliche Salze als Wirkstoff in Kombination mit üblichen pharmakologisch verträglichen Trägerstoffen und/oder Verdünnungsmitteln. Die Arzneimittel können oral oder parenteral verabreicht werden und liegen als Tabletten, Kapseln, Sirups, Trockenpulver, injizierbare und infundierbare Lösungen oder Suspensionen vor. Sie können aber auch als Suppositorien konfektioniert werden. Im allgemeinen werden oral verabreichbare Präparate bevorzugt.

Die Dosierung der erfindungsgemäßen Arzneipräparate hängt von verschiedenen Faktoren ab, wie von der Art und der Schwere der Krankheit oder von der verwendeten Verbindung. Im allgemeinen genügt bei oraler Gabe eine Einzeldosis von 1 bis 50, insbesondere 2 bis 20 mg, um zufriedenstellende Ergebnisse zu erhalten.

Beispiel I

Kapseln mit 10 mg 3-Methylamino-1-benzoxepin-5(2H)-on als Wirkstoff

Zusammensetzung :

Wirkstoff 10 Teile

| | |
|---|---|
| Lactose | 65 Teile |
| Maisstärke, getrocknet | 40 Teile |
| lösliche Stärke | 4 Teile |
| Magnesiumstearat | 1 Teil |
| | 120 Teile |

Herstellungsvorschrift :

Der Wirkstoff wird mit Lactose und Maisstärke vermischt. Die entstandene Mischung wird mit einer 15 %-igen wäßrigen Lösung der löslichen Stärke durchfeuchtet und granuliert. Die feuchte Masse wird durch ein 1,6 mm-Sieb passiert, bei 40 °C auf Horden getrocknet und anschließend durch ein 1,0 mm-Sieb passiert. Nach dem Vermischen des Granulats mit Magnesiumstearat wird die entstandene Mischung in Mengen von 120 mg verkapselt, so daß jede Kapsel 10 mg Wirkstoff enthält.

Die nachstehenden Beispiele dienen zur Erläuterung der Erfindung.

## Beispiel 1

Eine Lösung von 88 g (0,5 Mol) 2,3,4,5-Tetrahydro-1-benzoxepin-3,5-dion und einer Spatelspitze p-Toluolsulfonsäure in 750 ml Toluol wird unter Rühren mit 44 g (0,5 Mol) N,N-Dimethyläthylendiamin versetzt und anschließend bis zur Beendigung der Reaktion bei Raumtemperatur gerührt. Der nach Einengen der Lösung gewonnene Rückstand wird abgesaugt und aus Benzol/Ligroin umkristallisiert. Es werden 108 g (88 % d. Th.) 3-(β-Dimethylamino-äthylamino)-1-benzoxepin-5(2H)-on mit Fp. 100-101 °C erhalten.

## Beispiel 2

Eine Lösung von 17,6 g (0,1 Mol) 2,3,4,5-Tetrahydro-1-benzoxepin-3,5-dion und einer Spatelspitze p-Toluolsulfonsäure in 200 ml Dichlormethan wird unter Rühren mit 6 g (0,1 Mol) Isopropylamin versetzt und anschließend bis zur Beendigung der Reaktion bei Raumtemperatur gerührt. Der nach Einengen der Lösung gewonnene Rückstand wird abgesaugt und aus Butylacetat umkristallisiert. Es werden 13,5 g (62 % d. Th.) 3-Isopropylamino-1-benzoxepin-5(2H)-on mit Fp. 150-152 °C erhalten.

## Beispiel 3

In eine siedende Lösung von 52,8 g (0,3 Mol) 2,3,4,5-Tetrahydro-1-benzoxepin-3,5-dion und einer Spatelspitze p-Toluolsulfonsäure in 225 ml Toluol wird unter Rühren Dimethylamin eingeleitet. Das entstehende Wasser wird am Wasserabscheider abgetrennt. Nach beendeter Reaktion wird die Lösung eingeengt, der verbleibende Rückstand abgesaugt und aus Chloroform/Äther umkristallisiert. Es werden 42 g (69 % d. Th.) 3-Dimethylamino-1-benzoxepin-5(2H)-on mit Fp. 136-138 °C erhalten.

## Beispiel 4

In eine siedende Lösung von 160 g (0,9 Mol) 2,3,4,5-Tetrahydro-1-benzoxepin-3,5-dion und 1 ml Ameisensäure in 500 ml Dichlormethan wird unter Rühren Methylamin eingeleitet. Das entstehende Wasser wird am Wasserabscheider abgetrennt. Nach beendeter Reaktion wird die Lösung mit Eis gekühlt, das 3-Methylamino-1-benzoxepin-5(2H)-on abgesaugt und aus Methanol umkristallisiert. Es werden 140 g (81 % d. Th.) der Verbindung mit Fp. 176-178 °C erhalten.

## Beispiel 5

In eine siedende Lösung von 70,4 g (0,4 Mol) 2,3,4,5-Tetrahydro-1-benzoxepin-3,5-dion in 400 ml Chloroform wird unter Rühren Ammoniak eingeleitet. Das entstehende Wasser wird am Wasserabscheider abgetrennt. Nach beendeter Reaktion wird die Lösung abgekühlt, das 3-Amino-1-benzoxepin-5(2H)-on abgesaugt und umkristallisiert. Ausbeute 60,5 g (86 % d. Th.), Fp. 196-200 °C aus Chloroform.

## Beispiel 6

Zu einer Lösung von 35,2 g (0,2 Mol) 2,3,4,5-Tetrahydro-1-benzoxepin-3,5-dion in 200 ml Dichlormethan werden 38,1 g (0,3 Mol) Oxalylchlorid gegeben. Nach 14 h bei Raumtemperatur wird das Lösungsmittel abgezogen und das zurückbleibende Öl destilliert. Die bei 150-170 °C/3 mbar übergehende Fraktion, die in der Hauptsache aus 3-Chlor-1-benzoxepin-5(2H)-on besteht, wird in 100 ml Chloroform aufgenommen. Die erhaltene Lösung wird mit Eis gekühlt und tropfenweise unter Kühlung mit einem Überschuß von Piperidin, gelöst in Dichlormethan, versetzt. Bis zur Beeindigung der Reaktion wird bei 0 °C gerührt, die Reaktionslösung anschließend auf Eis gegossen und die organische Phase abgetrennt ; letztere wird mit Wasser gewaschen, getrocknet und eingedampft. Das 3-Piperidino-1-benzoxepin-5(2H)-on wird aus Äther umkristallisiert. Es werden 19,3 g (40 % d. Th., bezogen auf 2,3,4,5-Tetrahydro-1-benzoxepin-3,5-dion) mit Fp. 101-103 °C erhalten.

## Beispiel 7

Auf die in den Beispielen 1 bis 6 beschriebene Weise erhält man mit ähnlichen Ausbeuten aus

2,3,4,5-Tetrahydro-1-benzoxepin-3,5-dion und β,β-dimethyl-γ-dimethylamino-propylamin, n-Butylamin, Benzylamin, Morpholin, γ-Dimethylamino-propylamin, Phenäthylamin, Diäthylamin, Pyrrolidin, β-Methoxyäthylamin, N-Benzylpiperazin oder tert.-Butylamin

| | Fp. °C |
|---|---|
| 3-(β,β-Dimethyl-γ-dimethylamino-propylamino)-1-benzoxepin-5(2H)-on | 111-113 |
| 3-(n-Butylamino)-1-benzoxepin-5(2H)-on | 120-122 |
| 3-Benzylamino-1-benzoxepin-5(2H)-on | 157-160 |
| 3-Morpholino-1-benzoxepin-5(2H)-on | 126-129 |
| 3-(γ-Dimethylamino-propylamino)-1-benzoxepin-5(2H)-on | 118-120 |
| 3-Phenäthylamino-1-benzoxepin-5(2H)-on | 180-182 |
| 3-Diäthylamino-1-benzoxepin-5(2H)-on | 95-96 |
| 3-Pyrrolidino-1-benzoxepin-5(2H)-on | 118-122 |
| 3-(β-Methoxyäthylamino)-1-benzoxepin-5(2H)-on | 108-110 |
| 3-(N-Benzylpiperazino)-1-benzoxepin-5(2H)-on | 132-135 |
| 3-(tert.-Butylamino)-1-benzoxepin-5(2H)-on IR ($CH_2Cl_2$) 1 605 $cm^{-1}$ | Öl |

## Beispiel 8

Auf die in den Beispielen 1 bis 6 beschriebene Weise erhält man mit ähnlichen Ausbeuten aus

2,3,4,5-Tetrahydro-7-fluor-1-benzoxepin-3,5-dion,
2,3,4,5-Tetrahydro-7-nitro-1-benzoxepin-3,5-dion,
2,3,4,5-Tetrahydro-7,8-dichlor-1-benzoxepin-3,5-dion,
2,3,4,5-Tetrahydro-7,8-dimethyl-1-benzoxepin-3,5-dion,
2,3,4,5-Tetrahydro-7-brom-1-benzoxepin-3,5-dion,
2,3,4,5-Tetrahydro-7-methoxy-1-benzoxepin-3,5-dion,
2,3,4,5-Tetrahydro-8-methoxy-1-benzoxepin-3,5-dion,
2,3,4,5-Tetrahydro-7-chlor-1-benzoxepin-3,5-dion,
2,3,4,5-Tetrahydro-8-chlor-1-benzoxepin-3,5-dion,
2,3,4,5-Tetrahydro-7-methyl-1-benzoxepin-3,5-dion,
2,3,4,5-Tetrahydro-7-äthyl-1-benzoxepin-3,5-dion,
2,3,4,5-Tetrahydro-8-methyl-1-benzoxepin-3,5-dion,
2,3,4,5-Tetrahydro-7-chlor-8-methyl-1-benzoxepin-3,5-dion,
2,3,4,5-Tetrahydro-8-tert.-butyl-1-benzoxepin-3,5 dion und Methylamin, Dimethylamin oder γ-Dimethylaminopropylamin die Verbindungen

| | Fp. °C |
|---|---|
| 3-Methylamino-7-fluor-1-benzoxepin-5(2H)-on (0,25 $H_2O$) | 216-219 |
| 3-Methylamino-7-nitro-1-benzoxepin-5(2H)-on | 200 |
| 3-Methylamino-7,8-dichlor-1-benzoxepin-5(2H)-on | 238-241 |
| 3-Methylamino-7,8-dimethyl-1-benzoxepin-5(2H)-on | 218-222 |
| 3-Methylamino-7-brom-1-benzoxepin-5(2H)-on | 200-202 |
| 3-Methylamino-7-methoxy-1-benzoxepin-5(2H)-on | 169-172 |
| 3-Methylamino-8-methoxy-1-benzoxepin-5(2H)-on | 211-214 |
| 3-Methylamino-7-chlor-1-benzoxepin-5(2H)-on | 136-198 |
| 3-Methylamino-8-chlor-1-benzoxepin-5(2H)-on | 204-208 |
| 3-Methylamino-7-methyl-1-benzoxepin-5(2H)-on | 178-180 |
| 3-Methylamino-7-äthyl-1-benzoxepin-5(2H)-on | 181-183 |

| | Fp. °C |
|---|---|
| 3-Dimethylamino-8-chlor-1-benzoxepin-5(2H)-on | 126-129 |
| 3-(γ-Dimethylamino-propylamino)-7-chlor-1-benzoxepin-5(2H)-on-hydrochlorid | 206-208 |
| 3-Methylamino-7-chlor-8-methyl-1-benzoxepin-5(2H)-on | 229-234 |
| 3-Methylamino-8-methyl-1-benzoxepin-5(2H)-on | 182-184 |
| 3-Methylamino-8-tert.-butyl-1-benzoxepin-5(2H)-on | 195-196 |

Beispiel 9

Zu einer auf − 20 °C gekühlten Lösung von 242 g (1 Mol) (2'-Acetyl-4'-chlor)-phenoxyessigsäuremethylester in 300 ml Dimethylformamid werden 30,1 g (1 Mol) Natriumhydrid (80 % in Öl) unter Kühlung so in kleinen Portionen zugefügt, daß die Temperatur nicht über − 10 °C ansteigt. Anschließend wird noch 45 min bei −15 °C gerührt, dann die Lösung vorsichtig in Eiswasser gegossen und einmal mit Toluol extrahiert. Nach Ansäuern der wäßrigen Phase wird das ausgefallene Produkt abgesaugt und aus Cyclohexan/Toluol umkristallisiert. Es werden 126 g 2,3,4,5-Tetrahydro-7-chlor-1-benzoxepin-3,5-dion mit Fp. 131-134 °C erhalten (60 % d. Th.).

Beispiel 10

Zu einer Lösung von 28,7 g (0,1 Mol) (2'-Acetyl-4'-brom)-phenoxyessigsäure-methylester in 150 ml trockenem Tetrahydrofuran werden 8,8 g (0,11 Mol) Lithium-tert'-butylat in 50 ml trockenem Tetrahydrofuran unter Kühlung so zugetropft, daß die Temperatur zwischen 25-35 °C gehalten wird. Anschließend wird die Suspension in 400 ml Petroläther gegossen und das ausgefallene Lithiumsalz des 2,3,4,5-Tetrahydro-7-brom-1-benzoxepin-3,5-dion abgesaugt. Dieses Salz wird in ein Gemisch aus 150 ml Wasser und 11 ml Salzsäure (32 %) eingetragen. Das ausgefallene Produkt wird abgesaugt, in Dichlormethan gelöst, die Lösung mit gesättigter Kochsalzlösung gewaschen, über Natriumsulfat getrocknet, eingedampft und der Rückstand aus Cyclohexan umkristallisiert. Es wurden 11,7 g (46 % d. Th.) 2,3,4,5-Tetrahydro-7-brom-1-benzoxepin-3,5-dion mit Fp. 110-112 °C erhalten.

**Patentansprüche** (für die Vertragsstaaten : BE, CH, DE, FR, GB, IT, LI, LU, NL, SE)

1. 3-Amino-1-benzoxepin-5(2H)-on-Derivate der Formel I

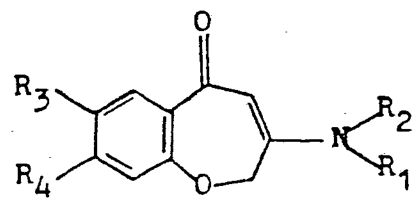

worin $R_1$ und $R_2$ unabhängig voneinander jeweils ein Wasserstoffatom, eine $C_1$-$C_5$-Alkylgruppe, welche gegebenenfalls einen unsubstituierten Phenylrest oder einen durch ein oder zwei Halogenatome, Methyl- oder Methoxygruppen oder eine 3,4-Methylendioxy- oder 3,4-Äthylendioxygruppe substituierten Phenylrest endständig tragen kann, eine gegebenenfalls endständig mit einer Hydroxy- oder Methoxygruppe substituierte $C_2$-$C_5$-Alkylgruppe oder eine $C_3$-$C_4$-Alkenylgruppe bedeuten, oder einer der beiden Reste $R_1$ und $R_2$ ein Wasserstoffatom oder eine $C_1$-$C_5$-Alkylgruppe und der andere eine $C_2$-$C_5$-Alkylgruppe, endständig substituiert mit einer $NR_5R_6$-Gruppe — in welcher $R_5$ und $R_6$ unabhängig voneinander jeweils ein Wasserstoffatom oder eine $C_1$-$C_5$-Alkylgruppe sind oder in welcher Alkylgruppen $R_5$ und $R_6$ direkt oder über ein Heteroatom O, S oder $NR_7$ unter Bildung eines 5- bis 7-Ringes miteinander verbunden sind — bedeuten, oder die Alkylgruppen $R_1$ und $R_2$ direkt oder über ein Heteroatom O, S oder $NR_7$ unter Bildung eines 5- bis 7-Ringes miteinander verbunden sind wobei $R_7$ ein Wasserstoffatom, eine Methyl-, Benzyl- oder Phenylgruppe ist, und worin $R_3$ und $R_4$ unabhängig voneinander jeweils Wasserstoffatom, Halogenatom, Alkyl-, Alkoxy- oder Alkylthiogruppe bedeuten, wobei der Alkylrest jeweils 1 bis 4 Kohlenstoffatome haben kann, oder einer der beiden Reste $R_3$ und $R_4$ Trifluormethyl- oder Nitrogruppe und der andere Wasserstoffatom sind, sowie Säureadditionssalze solcher Verbindungen der Formel I, worin $R_1$ und/oder $R_2$ ein basisches Stickstoffatom enthalten.

2. 3-A-1-benzoxepin-5(2H)-on, in welchem A bedeutet Amino, Methylamino, Isopropylamino, n-Butylamino, tert.-Butylamino, Benzylamino, Phenäthylamino, Diäthylamino, Pyrrolidino, Piperidino, Morpholino, N-Benzylpiperazino, β-Dimethylamino-äthylamino, β,β-Dimethyl-γ-dimethylaminopropylamino oder β-Methoxy-äthylamino.

3. 3-Methylamino-7-B-1-benzoxepin-5(2H)-on, in welchem B bedeutet Chlor, Brom, Methyl, Äthyl oder Methoxy.

4. 3-Methylamino-8-C-1-benzoxepin-5(2H)-on, in welchem C bedeutet Chlor, Methyl, tert.-Butyl, Methoxy.

5. 3-Methylamino-7,B,8-C-1-benzoxepin-5(2H)-on, in welchem B und C gleich oder verschieden sind und Chlor und Methyl bedeuten.

6. 3-(γ-Dimethylaminopropylamino)-7-B-1-benzoxepin-5(2H)-on, in welchem B Wasserstoff oder Chlor bedeutet.

7. 3-Dimethylamino-8-C-1-benzoxepin-5(2H)-on, in welchem C Wasserstoff oder Chlor bedeutet.

8. Verfahren zur Herstellung von 3-Amino-1-benzoxepin-5(2H)-on-Derivaten der Formel I gemäss

Anspruch 1, sowie von Säureadditionssalzen solcher Verbindungen der Formel I, worin $R_1$ und/oder $R_2$ ein basisches Stickstoffatom enthalten, dadurch gekennzeichnet, daß man eine Verbindung der Formel II

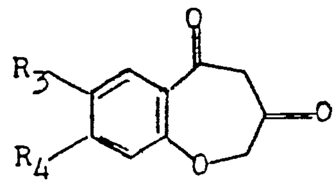

worin $R_3$ und $R_4$ die obige Bedeutung haben, oder eine Verbindung der Formel IV

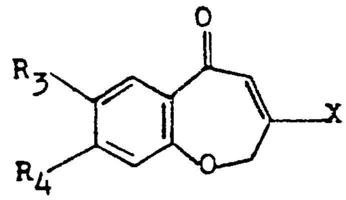

worin $R_3$ und $R_4$ die obige Bedeutung haben und X Chlor oder Brom ist, mit einem Amin der Formel III

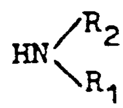

worin $R_1$ und $R_2$ die obige Bedeutung haben, in einem inerten Lösungsmittel umsetzt, die freie Base isoliert und solche Verbindungen der Formel I, worin $R_1$ und/oder $R_2$ ein basisches Stickstoffatom enthalten, gegebenenfalls in ein Säureadditionssalz überführt oder aus deren Säureadditionssalz die freie Base isoliert.

**Patentansprüche** (für den Vertragsstaat AT)

1. Verfahren zur Herstellung von 3-Amino-1-benzoxepin-5(2H)-on-Derivaten der Formel I

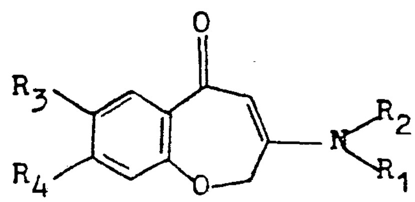

worin $R_1$ und $R_2$ unabhängig voneinander jeweils ein Wasserstoffatom, eine $C_1$-$C_5$-Alkylgruppe, welche gegebenenfalls einen unsubstituierten Phenylrest oder einen durch ein oder zwei Halogenatome, Methyl- oder Methoxygruppen oder eine 3,4-Methylendioxy- oder 3,4-Äthylendioxygruppe substituierten Phenylrest endständig tragen kann, eine gegebenfalls endständig mit einer Hydroxy- oder Methoxygruppe substituierte $C_2$-$C_5$-Alkylgruppe oder eine $C_3$-$C_4$-Alkylengruppe bedeuten, oder einer der beiden Reste $R_1$ und $R_2$ ein Wasserstoffatom oder eine $C_1$-$C_5$-Alkylgruppe und der andere eine $C_2$-$C_5$-Alkylgruppe, endständig substituiert mit einer $NR_5R_6$-Gruppe — in welcher $R_5$ und $R_6$ unabhängig voneinander jeweils ein Wasserstoffatom oder eine $C_1$-$C_5$-Alkylgruppe sind oder in welcher Alkylgruppen $R_5$ und $R_6$ direkt oder über ein Heteroatom O, S oder $NR_7$ unter Bildung eines 5- bis 7-Ringes miteinander verbunden sind — bedeuten, oder die Alkylgruppen $R_1$ und $R_2$ direkt oder über ein Heteroatom O, S oder $NR_7$ unter Bildung eines 5- bis 7-Ringes miteinander verbunden sind, wobei $R_7$ ein Wasserstoffatom, eine Methyl-, Benzyl- oder Phenylgruppe ist, und worin $R_3$ und $R_4$ unabhängig voneinander jeweils Wasserstoffatom, Halogenatom, Alkyl-, Alkoxy- oder Alkylthiogruppe bedeuten, wobei der Alkylrest jeweils 1 bis 4 Kohlenstoffatome haben kann, oder einer der beiden Reste $R_3$ und $R_4$ Trifluormethyl- oder Nitrogruppe und der andere Wasserstoffatom sind, sowie von Säureadditionssalzen solcher Verbindungen der Formel I, worin $R_1$ und/oder $R_2$ ein basisches Stickstoffatom enthalten, dadurch gekennzeichnet, daß man eine Verbindung der Formel II

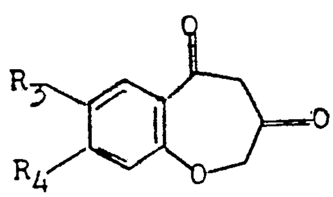

worin $R_3$ und $R_4$ die obige Bedeutung haben, oder eine Verbindung der Formel IV

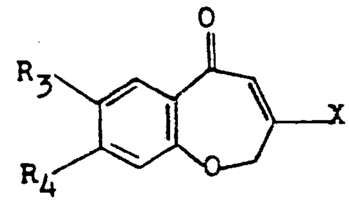

worin $R_3$ und $R_4$ die obige Bedeutung haben und X Chlor oder Brom ist, mit einem Amin der Formel III

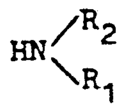

worin $R_1$ und $R_2$ die obige Bedeutung haben, in einem inerten Lösungsmittel umsetzt, die freie Base isoliert und solche Verbindungen der Formel I, worin $R_1$ und/oder $R_2$ ein basisches Stickstoffatom enthalten, gegebenenfalls in ein Säureadditionsalz überführt oder aus deren Säureadditionssalz die freie Base isoliert.

2. Verfahren gemäß Anspruch 1, dadurch gekennzeichnet, daß 3-A-1-benzoxepin-5(2H)-one hergestellt werden, in welchen A bedeutet Amino, Methylamino, Isopropylamino, n-Butylamino, tert.-Butylamino, Benzylamino, Phenäthylamino, Diäthylamino, Pyrrolidino, Piperidino, Morpholino, N-Benzylpiperazino, β-Dimethylamino-äthylamino, β,β-Dimethyl-γ-dimethylaminopropylamino oder β-Methoxyäthylamino.

3. Verfahren gemäß Anspruch 1, dadurch gekennzeichnet, daß 3-Methylamino-7-B-1-benzoxepin-5(2H)-one hergestellt werden, in welchen B bedeutet Chlor, Brom, Methyl, Äthyl oder Methoxy.

4. Verfahren gemäß Anspruch 1, dadurch gekennzeichnet, daß 3-Methylamino-8-C-1-benzoxepin-5(2H)-one hergestellt werden, in welchen C bedeutet Chlor, Methyl, tert.-Butyl, Methoxy.

5. Verfahren gemäß Anspruch 1, dadurch gekennzeichnet, daß 3-Methylamino-7-B, 8-C-1-benzoxepin-5(2H)-one hergestellt werden, in welchen B und C gleich oder verschieden sind und Chlor und Methyl bedeuten.

6. Verfahren gemäß Anspruch 1, dadurch gekennzeichnet, daß 3-(γ-Dimethylaminopropylamino)-7-B-1-benzoxepin-5(2H)-one hergestellt werden, in welchen B Wasserstoff oder Chlor bedeutet.

7. Verfahren gemäß Anspruch 1, dadurch gekennzeichnet, daß 3-Dimethylamino-8-C-1-benzoxepin-5(2H)-one hergestellt werden, in welchen C Wasserstoff oder Chlor bedeutet.

**Claims** (for the Contracting States : BE, CH, DE, FR, GB, IT, LI, LU, NL, SE)

1. 3-Amino-1-benzoxepin-5(2H)-one derivatives of the Formula I

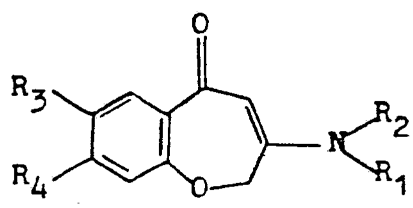

where $R_1$ and $R_2$ independently of one another each denote a hydrogen atom, a $C_1$-$C_5$-alkyl group, which may optionally carry at the end an unsubstituted phenyl radical or a phenyl radical which is substituted with one or two halogen atoms, methyl or methoxy groups or one 3,4-methylenedioxy or 3,4-ethylenedioxy group, a $C_2$-$C_5$-alkyl group which is optionally end-substituted with a hydroxy group or methoxy group, or a $C_3$-$C_4$-alkenyl group, or one of the two radicals $R_1$ and $R_2$ denotes a hydrogen atom or a $C_1$-$C_5$-alkyl group and the other denotes a $C_2$-$C_5$-alkyl group which is end-substituted with an $NR_5R_6$ group, in which $R_5$ and $R_6$ independently of one another are in each case a hydrogen atom or a $C_1$-$C_5$-alkyl group, or in which alkyl groups $R_5$ and $R_6$ are linked to one another directly or via a hetero-atom O, S or $NR_7$ forming a 5-membered to 7-membered ring, or the alkyl groups $R_1$ and $R_2$ are linked to one another directly or via a hetero-atom O, S or $NR_7$ forming a 5-membered to 7-membered ring, where $R_7$ is a hydrogen atom, a methyl, benzyl or phenyl group and where $R_3$ and $R_4$ independently of one another each denote a hydrogen atom, a halogen atom, an alkyl, alkoxy or alkylthio group, in which each alkyl radical may have 1 to 4 carbon atoms, or one of the two radicals $R_3$ and $R_4$ is a trifluoromethyl group or nitro group and the other is a hydrogen atom, and acid addition salts of those compounds of Formula I in which $R_1$ and/or $R_2$ contain(s) a basic nitrogen atom.

2. 3-A-1-benzoxepin-5(2H)-one, in which A denotes amino, methylamino, isopropylamino, n-butylamino, tert.-butylamino, benzylamino, phenethylamino, diethylamino, pyrrolidino, piperidino, morpholino, N-benzyl-piperazino, β-dimethyl-amino-ethylamino, β,β-dimethyl-γ-dimethylaminopropylamino or β-methoxy-ethylamino.

3. 3-Methylamino-7-B-1-benzoxepin-5(2H)-one, in which B denotes chlorine, bromine, methyl, ethyl or methoxy.

4. 3-Methylamino-8-C-1-benzoxepin-5(2H)-one, in which C denotes chlorine, methyl, tert. butyl, methoxy.

5. 3-Methylamino-7-B,8-C-1-benzoxepin-5(2H)-one, in which B and C are identical or different and denote chlorine and methyl.

6. 3-(γ-Dimethylaminopropylamino)-7-B-1-benzoxepin-5(2H)-one, in which B denotes hydrogen or chlorine.

7. 3-Dimethylamino-8-C-1-benzoxepin-5(2H)-one, in which C denotes hydrogen or chlorine.

8. Process for the preparation of 3-amino-l-benzoxepin-5(2H)-one derivatives of the Formula I according to claim 1, and of acid addition salts of those compounds of Formula I in which $R_1$ and/or $R_2$ contain(s) a basic nitrogen atom, characterized in that a compound of Formula II

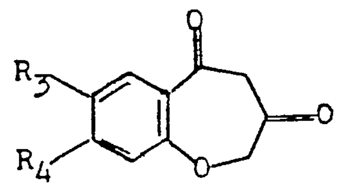

where $R_3$ and $R_4$ have the above meanings, or a compound of the Formula IV

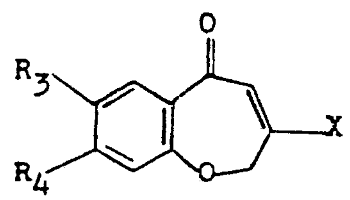

where $R_3$ and $R_4$ have the above meanings and X is chlorine or bromine, is reacted with an amine of the Formula III

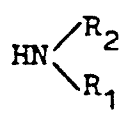

where $R_1$ and $R_2$ have the above meanings, in an inert solvent, the free base is isolated and for a compound of Formula I in which $R_1$ and/or $R_2$ contain(s) a basic nitrogen atom, is optionally converted into an acid addition salt, or the free base is isolated from the acid addition salt thereof.

**Claims** (for the Contracting State AT)

1. Process for the preparation of 3-amino-1-benzoxepin-5(2H)-one derivatives of the Formula I

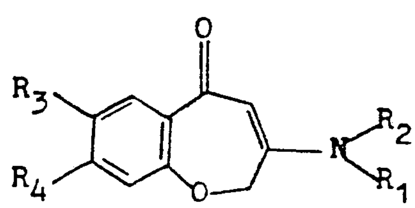

where $R_1$ and $R_2$ independently of one another each denote a hydrogen atom, a $C_1$-$C_5$-alkyl group, which may optionally carry at the end an unsubstituted phenyl radical or a phenyl radical which is substituted with one or two halogen atoms, methyl or methoxy groups or one 3,4-methylenedioxy or 3,4-ethylenedioxy group, a $C_2$-$C_5$-alkyl group which is optionally end-substituted with a hydroxy group or methoxy group, or a $C_3$-$C_4$-alkenyl group, or one of the two radicals $R_1$ and $R_2$ denotes a hydrogen atom or a $C_1$-$C_5$-alkyl group and the other denotes a $C_2$-$C_5$-alkyl group which is end-substituted with and $NR_5R_6$ group, in which $R_5$ and $R_6$ independently of one another are in each case a hydrogen atom or a

$C_1$-$C_5$-alkyl group, or in which alkyl groups $R_5$ and $R_6$ are linked to one another directly or via a hetero-atom O, S or $NR_7$ forming a 5-membered to 7-membered ring, or the alkyl groups $R_1$ and $R_2$ are linked to one another directly or via a hetero-atom O, S or $NR_7$ forming a 5-membered to 7-membered ring, where $R_7$ is a hydrogen atom, a methyl, benzyl or phenyl group and where $R_3$ and $R_4$ independently of one another each denote a hydrogen atom, a halogen atom, an alkyl, alkoxy or alkylthio group, in which each alkyl radical may have 1 to 4 carbon atoms, or one of the two radicals $R_3$ and $R_4$ is a trifluoromethyl group or nitro group and the other is a hydrogen atom, and acid addition salts of those compounds of Formula I in which $R_1$ and/or $R_2$ contain(s) a basic nitrogen atom characterized in that a compound of Formula II

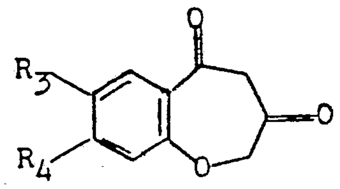

where $R_3$ and $R_4$ have the above meanings, or a compound of the Formula IV

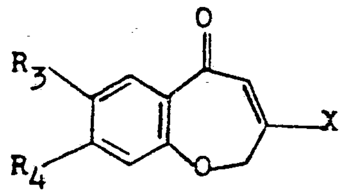

where $R_3$ and $R_4$ have the above meanings and X is chlorine or bromine, is reacted with an amine of the Formula III

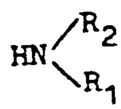

where $R_1$ and $R_2$ have the above meanings, in an inert sovlent, the free base is isolated and for a compound of Formula I in which $R_1$ and/or $R_2$ contain(s) a basic nitrogen atom, is optionally converted into an acid addition salt, or the free base is isolated from the acid addition salt thereof.

2. Process according to claim 1, characterized in that 3-A-1-benzoxepin-5(2H)-one, in which A denotes amino, methylamino, isopropylamino, n-butylamino, tert.-butylamino, benzylamino, phenethylamino, diethylamino, pyrrolidino, piperidino, morpholino, N-benzyl-piperazino, β-dimethyl-amino-ethylamino, β,β-dimethyl-γ-dimethylaminopropylamino or β-methoxy-ethylamino.

3. Process according to claim 1, characterized in that 3-methylamino-7-B-1-benzoxepin-5(2H)-one, in which B denotes chlorine, bromine, methyl, ethyl or methoxy.

4. Process according to claim 1, characterized in that 3-methylamino-8-C-1-benzoxepin-5(2H)-one, in which C denotes chlorine, methyl, tert. butyl, methoxy.

5. Process according to claim 1, characterized in that 3-methylamino-7-B,8-C-1-benzoxepin-5(2H)-one, in which B and C are identical or different and denote chlorine and methyl.

6. Process according to claim 1, characterized in that 3-(γ-dimethylaminopropylamino)-7-B-1-benzoxepin-5(2H)-one, in which B denotes hydrogen or chlorine.

7. Process according to claim 1, characterized in that 3-dimethylamino-8-C-1-benzoxepin-5(2H)-one, in which C denotes hydrogen or chlorine.

**Revendications** (pour les Etats contractants : BE, CH, DE, FR, GB, IT, LI, LU, NL, SE)

1. Dérivés de (2H)-amino-3 benzoxépinne-1 ones-5 de formule I

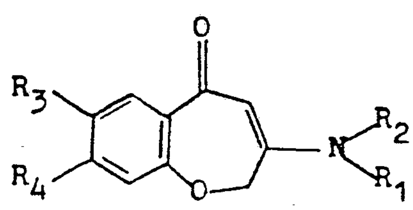

dans laquelle $R_1$ et $R_2$, indépendamment l'un de l'autre, représentent chacun un atome d'hydrogène, un

groupe alkyle en $C_1$-$C_5$, qui peut porter éventuellement à son extrémité un radical phényle non substitué ou un radical phényle substitué par un ou deux atomes d'halogène, groupes méthyles ou méthoxy, ou par un groupe méthylènedioxy-3,4 ou éthylènedioxy-3,4, un groupe alkyle en $C_2$-$C_5$ éventuellement substitué à son extrémité par un groupe hydroxy ou méthoxy ou un groupe alcényle en $C_3$-$C_4$ ou bien l'un des deux radicaux, $R_1$ et $R_2$ désignent un atome d'hydrogène ou un groupe alkyle en $C_1$-$C_5$ et l'autre désigne un groupe alkyle en $C_1$-$C_5$ et l'autre désigne un groupe alkyle $C_2$-$C_5$, substitué à son extrémité par un groupe $NR_5R_6$ — dans lequel $R_5$ et $R_6$, indépendamment l'un de l'autre, sont chacun un atome d'hydrogène ou un groupe alkyle en $C_1$-$C_5$ ou dans lequel les groupes alkyles $R_5$ et $R_6$ sont liés entre eux directement ou par l'intermédiaire d'un hétéroatome O, S ou de $NR_7$ avec formation d'un cycle à 5 à 7 atomes — ou bien les groupes alkyles $R_1$ et $R_2$ sont liés entre eux directement ou par l'intermédiaire d'un hétéroatome O, S ou de $NR_7$ avec formation d'un cycle de 5 à 7 atomes, $R_7$ désignant un atome d'hydrogène, un groupe méthyle, benzyle ou phényle et dans laquelle $R_3$ et $R_4$, indépendamment l'un de l'autre, représentent chacun un atome d'hydrogène, un atome d'halogène, un groupe alkyle, alcoxy ou alkylthio, le groupe alkyle pouvant avoir 1 à 4 atomes de carbone, ou bien un des deux radicaux $R_3$ et $R_4$ est un groupe trifluorométhyle ou nitro et l'autre est un atome d'hydrogène, ainsi que les sels d'addition d'un acide de ces composés de formule I, dans laquelle $R_1$ et/ou $R_2$ contiennent un atome d'azote basique.

2. (2H)-A-3 benzoxépinne-1 one-5 dans laquelle A désigne un groupe amino, méthylamino, isopropylamino, n-butylamino, tert-butylamino, benzylamino, phénéthylamino, diéthylamino, pyrrolidino, pipéridino, morpholino, N-benzylpipérazino, β-diméthylamino-éthylamino, β,β-diméthyl-γ-diméthylaminopropylamino ou β-méthoxyéthylamino.

3. (2H)-méthylamino-3 B-7 benzoxépinne-1 one-5, dans laquelle B désigne le chlore, le brome, un groupe méthyle, éthyle ou méthoxy.

4. (2H)-méthylamino-3 C-8 benzoxépinne-1 one-5, dans laquelle C désigne le chlore, un groupe méthyle, tertiobutyle, méthoxy.

5. (2H)-méthylamino-3 B-7, C-8 benzoxépinne-1 one-5, dans laquelle B et C sont égaux ou différents et désignent le chlore ou le groupe méthyle.

6. (2H)-γ-diméthylaminopropylamino-3-B-7 benzoxépinne-1 one-5 dans laquelle B désigne l'hydrogène ou le chlore.

7. (2H)-diméthylamino-3 C-8 benzoxépinne-1 one-5 dans laquelle C désignel'hydrogène ou le chlore.

8. Procédé de préparation de dérivé de (2H)-amino-3 benzoxépinne-1 one-5 de formule I selon la revendication 1 et de sels d'addition d'acides de ces composés de formule I dans laquelle $R_1$ et/ou $R_2$ contiennent un atome d'azote basique, caractérisé en ce que l'on fait réagir dans un solvant inerte un composé de formule II

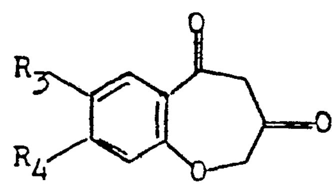

dans laquelle $R_3$ et $R_4$ ont la signification ci-dessus, ou un composé de formule IV

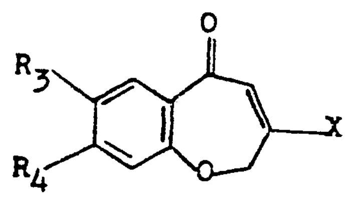

dans laquelle $R_3$ et $R_4$ ont la signification ci-dessus et X est le chlore ou le brome avec une amine de formule III

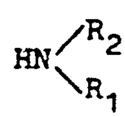

dans laquelle $R_1$ et $R_2$ ont la signification ci-dessus, on isole la base libre et on transforme éventuellement ces composés de formule I, dans laquelle $R_1$ et/ou $R_2$ contiennent un atome d'azote basique en un sel d'addition d'acide ou on isole la base libre à partir de son sel d'addition d'acide.

**Revendications** (pour l'Etat contractant AT)

1. Procédé de préparation de dérivé de (2H)-amino-3 benzoxépinne-1 ones-5 de formule I

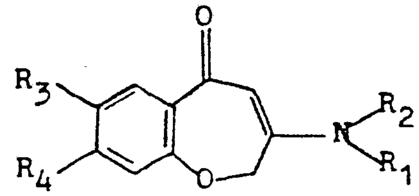

dans laquelle $R_1$ et $R_2$, indépendamment l'un de l'autre, représentent chacun un atome d'hydrogène, un groupe alkyle en $C_1$-$C_5$, qui peut porter éventuellement à son extrémité un radical phényle non substitué ou un radical phényle substitué par un ou deux atomes d'halogène, groupes méthyles ou méthoxy, ou par un groupe méthylènedioxy-3,4 ou éthylènedioxy-3,4 un groupe alkyle en $C_2$-$C_5$ éventuellement substitué à son extrémité par un groupe hydroxy ou méthoxy ou un groupe alcényle en $C_3$-$C_4$ ou bien l'un des deux radicaux, $R_1$ et $R_2$ désignent un atome d'hydrogène ou un groupe alkyle en $C_1$-$C_5$ et l'autre désigne un groupe alkyle $C_2$-$C_5$, substitué à son extrémité par un groupe $NR_5R_6$ — dans lequel $R_5$ et $R_6$, indépendamment l'un de l'autre, sont chacun un atome d'hydrogène ou un groupe alkyle en $C_1$-$C_5$ ou dans lequel les groupes alkyles $R_5$ et $R_6$ sont liés entre eux directement ou par l'intermédiaire d'un hétéroatome O, S ou de $NR_7$ avec formation d'un cycle à 5 à 7 atomes — ou bien les groupes alkyles $R_1$ et $R_2$ sont liés entre eux directement ou par l'intermédiaire d'un hétéroatome O, S ou de $NR_7$ avec formation d'un cycle de 5 à 7 atomes, $R_7$ désignant un atome d'hydrogène, un groupe méthyle, benzyle ou phényle et dans laquelle $R_3$ et $R_4$, indépendamment l'un de l'autre, représentent chacun un atome d'hydrogène, un atome d'halogène, un groupe alkyle, alcoxy ou alkylthio, le groupe alkyle pouvant avoir 1 à 4 atomes de carbone, ou bien un des deux radicaux $R_3$ et $R_4$ est un groupe trifluorométhyle ou nitro et l'autre est un atome d'hydrogène, ainsi que des sels d'addition d'un acide de ces composés de formule I, dans laquelle $R_1$ et/ou $R_2$ contiennent un atome d'azote basique, caractérisé en ce que l'on fait réagir dans un solvant inerte un composé de formule II

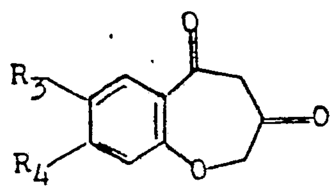

dans laquelle $R_3$ et $R_4$ ont la signification ci-dessus, ou un composé de formule IV

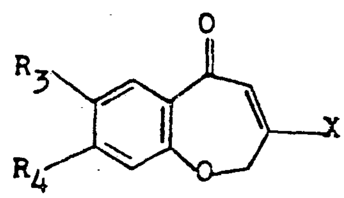

dans laquelle $R_3$ et $R_4$ ont la signification ci-dessus et X est le chlore ou le brome avec une amine de formule III

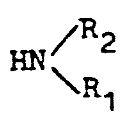

dans laquelle $R_1$ et $R_2$ ont la signification ci-dessus, on isole la base libre et on transforme éventuellement ces composés de formule I, dans laquelle $R_1$ et/ou $R_2$ contiennent un atome d'azote basique, en un sel d'addition d'acide ou on isole la base libre à partir de son sel d'addition d'acide.

2. Procédé selon la revendication 1, caractérisé en ce que l'on prépare des (2H)-A-3 benzoxépinne-1 ones-5 dans lesquelles A désigne un groupe amino, méthylamino, isopropylamino, n-butylamino, tert-butylamino, benzylamino, phénéthylamino, diéthylamino, pyrrolidino, pipéridino, morpholino, N-benzylpipérazino, β-diméthylamino-éthylamino, β,β-diméthyl-γ-diméthylaminopropylamino ou β-méthoxy-éthylamino.

3. Procédé selon la revendication 1, caractérisé en ce que l'on prépare des (2H)-méthylamino-3 B-7

benzoxépinne-1-ones-5 dans lesquelles B désigne le chlore, le brome, un groupe méthyle, éthyle ou méthoxy.

4. Procédé selon la revendication 1 caractérisé en ce que l'on prépare des (2H)-méthylamino-3 C-8 benzoxépinne-1 ones-5 dans lesquelles C désigne le chlore, un groupe méthyle, tertio-butyle, méthoxy.

5. Procédé selon la revendication 1 caractérisé en ce que l'on prépare des (2H)-méthylamino-3 B-7, C-8 benzoxépinne-1 ones-5 dans lesquelles B et C sont égaux ou différents et désignent le chlore ou le groupe méthyle.

6. Procédé selon la revendication 1 caractérisé en ce que l'on prépare des (2H)-γ-diméthylaminopropylamino-3 B-7 benzoxépinne-1 ones-5 dans lesquelles B désigne l'hydrogène ou le chlore.

7. Procédé selon la revendication 1 caractérisé en ce que l'on prépare des (2H)-diméthylamino-3 8-C benzoxépinne-1 ones-5 dans lesquelles C désigne l'hydrogène ou le chlore.